(19) Europäisches Patentamt / European Patent Office / Office européen des brevets

(11) **EP 4 356 836 A1**

(12) # EUROPEAN PATENT APPLICATION
published in accordance with Art. 153(4) EPC

(43) Date of publication:
**24.04.2024 Bulletin 2024/17**

(21) Application number: **22824471.1**

(22) Date of filing: **17.01.2022**

(51) International Patent Classification (IPC):
***A61B 6/00*** (2006.01)

(52) Cooperative Patent Classification (CPC):
**A61B 6/032; A61B 6/4014; A61B 6/5211;
A61B 6/5258; A61N 5/1049; A61N 5/1067;**
A61B 6/4085; A61N 2005/1061

(86) International application number:
**PCT/JP2022/001472**

(87) International publication number:
**WO 2022/264468 (22.12.2022 Gazette 2022/51)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **18.06.2021 JP 2021101326**

(71) Applicant: **Hitachi, Ltd.
Tokyo 100-8280 (JP)**

(72) Inventors:
• **KAGEYAMA Masahiro
Tokyo 100-8280 (JP)**
• **BABA Rika
Tokyo 100-8280 (JP)**
• **MATSUNO Motoki
Tokyo 100-8280 (JP)**
• **YOSHIDA Mitsuhiro
Tokyo 100-8280 (JP)**

(74) Representative: **MERH-IP Matias Erny Reichl
Hoffmann
Patentanwälte PartG mbB
Paul-Heyse-Strasse 29
80336 München (DE)**

(54) **RADIOLOGICAL IMAGE PROCESSING DEVICE, IMAGE PROCESSING METHOD, AND SYSTEM**

(57)    A radiation image processing apparatus for executing image processing on an image obtained by emitting radiation to an object, executes, in order to improve a contrast of the image, processing of extracting a low-frequency component of an input signal of the image to generate a first signal of the image, processing of dividing the input signal of the image by the first signal of the image to generate a second signal of the image, processing of executing non-linear processing on the second signal of the image to generate a third signal of the image, and processing of outputting an output signal of the image obtained by multiplying the input signal of the image by the third signal of the image.

[FIG. 7]

EP 4 356 836 A1

**Description**

Incorporation by Reference

The present application claims priority from Japanese Patent Application No. 2021-101326 filed on June 18, 2021, contents of which are incorporated into the present application by reference.

Technical Field

The present invention relates to a radiation image processing apparatus, an image processing method, and a system for performing therapy using a radiation image obtained based on radiation that transmits through an object.

Background Art

In therapy using radiation such as X-rays performed at a medical site, image guided radio therapy (IGRT), adaptive radio therapy (ART), and the like are important for realizing minimally invasive and economical therapy. In order to improve the accuracy and efficiency of the therapy, there is a large need to improve the image quality of a captured radiation image using computed tomography (CT) or the like.

A radiation image obtained by using a human body (patient) as an object is an image in which a difference in radiation transmittance among a bone, an internal organ, an organ, and tissue constituting the human body is used and the difference appears as a luminance difference of a shade. Therefore, a part at which the radiation transmittance is significantly different from that of a surrounding region, such as the bone, a lung, a thick blood vessel, a trachea, a solid nodule which is a kind of a cancer nodule, and the like, appears to have a high shade contrast in a radiation image. On the other hand, at soft tissue such as the internal organ such as a bladder and a prostate gland, and a ground-glass nodule which is a kind of the cancer nodule, since the difference in the radiation transmittance with that of the surrounding region thereof is relatively small, the shade contrast at the soft tissue is low and the visibility is poor in the radiation image.

In fields of natural images, many techniques for improving a contrast of the image by signal processing have been proposed, and for example, a contour enhancement technique called unsharp mask is known. However, in a general unsharp mask, since an image processing filter having a linear characteristic is used, a problem is known that when a thin contour of a soft tissue or the like is emphasized to be clear, a dark contour that originally has a large luminance difference with the surrounding will be overemphasized, resulting in an unnatural image.

Therefore, NPL 1 discloses a technique for improving a contrast of an entire image by emphasizing a thin contour of a distant view region or the like on which haze is applied while reducing enhancement of a dark contour by using a signal processing technique for greatly changing a luminance value using non-linear processing.

On the other hand, in a radiation image used for a therapy plan and the like, since the luminance value affects a radiation dose calculation (CT value), there is a constraint that an original luminance value needs to be maintained as much as possible. Therefore, a technique for improving the contrast by emphasizing only a contour of a shade (boundary with the surrounding) in the radiation image and not changing a luminance value of a flat region (for example, see PTL 1) is known.

An image processing apparatus described in PTL 1 obtains a blurred image signal Sus from an original image signal Sorg, subtracts the blurred image signal Sus from the original image signal Sorg to obtain a signal Sorg-Sus, converts the signal Sorg-Sus by using a non-linear conversion function f in which a value after the conversion decreases as the value increases to obtain a conversion signal f(Sorg-Sus), multiplies the conversion signal f(Sorg-Sus) by an improvement factor β(Sorg), and then subtracts the product from the original image signal Sorg. That is, PTL 1 discloses that a calculation as expressed in Equation (1) is performed.

[Math. 1]

$$Sproc = Sorg - \beta(Sorg) \times (Sorg - Sus) \dots (1)$$

Citation List

Patent Literature

PTL 1: JP2002-74356A

Non Patent Literature

NPL 1: Guang Deng; "A Generalized Unsharp Masking Algorithm", IEEE TRANSACTIONS ON IMAGE PROCESSING, VOL. 20, NO. 5, MAY 2011

Summary of Invention

Technical Problem

In the technique described in PTL 1, in a case of a shade of a region overlapping with a region at which the X-ray transmittance is low (hereinafter, referred to as a low transmittance region) such as a "bone", for example, a spine and a pelvis, the conversion signal f(Sorg-Sus) is small. Therefore, there is a problem that a contrast improvement effect for the shade is smaller than a contrast improvement effect for a shade of a region not overlapping with the low transmittance region.

An object of the invention is to provide an image processing technique for favorably improving a contrast of an image regardless of the presence or absence of overlapping with the low transmittance region.

Solution to Problem

Typical ones of the invention described in the present application will be briefly described as follows. That is, a radiation image processing apparatus for executing image processing on an image obtained by emitting radiation to an object, includes a calculation device, a storage device connected to the calculation device, and an interface connected to the calculation device. The radiation image processing apparatus acquires an input signal of the image, and executes contrast improvement processing on the input signal of the image to improve a contrast of the image. The contrast improvement processing includes processing of extracting a low-frequency component of the input signal of the image to generate a first signal of the image, processing of dividing the input signal of the image by the first signal of the image to generate a second signal of the image, processing of executing non-linear processing on the second signal of the image to generate a third signal of the image, and processing of outputting an output signal of the image obtained by multiplying the input signal of the image by the third signal of the image.

Advantageous Effects of Invention

According to the invention, a contrast of an image can be favorably improved regardless of the presence or absence of overlapping with a low transmittance region. Problems, configurations and effects other than those described above will be clarified by description of the following embodiment.

Brief Description of Drawings

    [FIG. 1] FIG. 1 is a diagram illustrating an example of a configuration of a radiation therapy system according to Embodiment 1.
    [FIG. 2] FIG. 2 is a diagram illustrating an example of a configuration of a radiation therapy apparatus according to Embodiment 1.
    [FIG. 3A] FIG. 3A is a diagram illustrating an example of a radiation image acquired by the radiation therapy apparatus according to Embodiment 1.
    [FIG. 3B] FIG. 3B is a diagram illustrating an example of a radiation image acquired by the radiation therapy apparatus according to Embodiment 1.
    [FIG. 4A] FIG. 4A is a diagram illustrating an example of a radiation image acquired by the radiation therapy apparatus according to Embodiment 1.
    [FIG. 4B] FIG. 4B is a diagram illustrating an example of a radiation image acquired by the radiation therapy apparatus according to Embodiment 1.
    [FIG. 5] FIG. 5 is a diagram illustrating an example of a configuration of a therapy plan apparatus according to Embodiment 1.
    [FIG. 6] FIG. 6 is a diagram illustrating an example of a functional configuration of an image processing unit according to Embodiment 1.
    [FIG. 7] FIG. 7 is a diagram illustrating an example of a configuration of a contrast improvement unit according to Embodiment 1.

[FIG. 8] FIG. 8 is a flowchart illustrating an example of processing executed by the contrast improvement unit according to Embodiment 1.

[FIG. 9] FIG. 9 is a diagram illustrating examples of non-linear processing executed by a non-linear calculation unit according to Embodiment 1.

[FIG. 10] FIG. 10 is a diagram illustrating examples of the non-linear processing executed by the non-linear calculation unit according to Embodiment 1.

[FIG. 11] FIG. 11 is a diagram illustrating an example of the non-linear processing executed by the non-linear calculation unit according to Embodiment 1.

[FIG. 12] FIG. 12 is a diagram illustrating an example of a result of signal processing executed by the contrast improvement unit according to Embodiment 1.

[FIG. 13] FIG. 13 is a diagram illustrating a configuration of an image processing apparatus described in PTL 1.

[FIG. 14] FIG. 14 is a diagram illustrating an example of a result of signal processing executed by the image processing apparatus described in PTL 1.

Description of Embodiments

An embodiment of the invention will be described below with reference to drawings. However, the invention should not be construed as being limited to the description of the embodiment described below. A person skilled in the art could have easily understood that specific configuration can be changed without departing from a spirit or a gist of the invention.

In configurations of the invention to be described below, the same or similar configurations or functions are denoted by the same reference numerals, and repeated descriptions thereof are omitted.

In order to facilitate understanding of the invention, a position, a size, a shape, a range, etc. of each component illustrated in the drawings may not represent an actual position, size, shape, range, etc. Therefore, the invention is not limited to the position, the size, the shape, the range, and the like disclosed in the drawings and the like.

Further, the embodiment of the invention may be implemented by hardware, software running on a general-purpose computer, or a combination of the hardware and the software.

[Embodiment 1]

<Configuration Example of Radiation Therapy System>

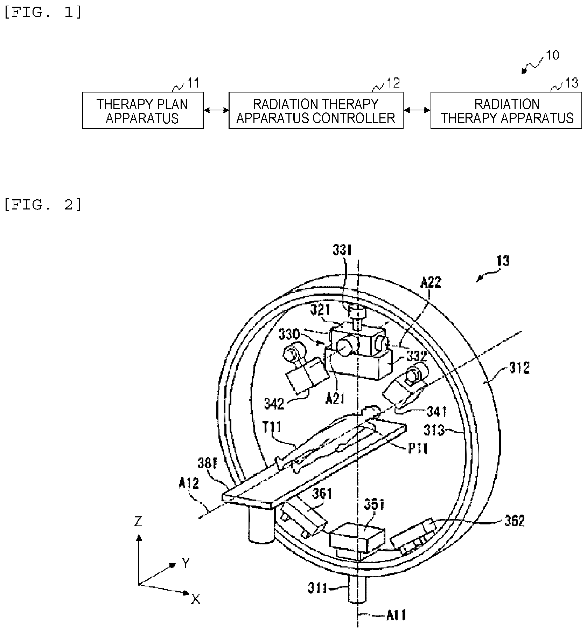

FIG. 1 is a diagram illustrating an example of a configuration of a radiation therapy system according to Embodiment 1.

A radiation therapy system 10 includes a therapy plan apparatus 11, a radiation therapy apparatus controller 12, and a radiation therapy apparatus 13.

The radiation therapy system 10 is a system for performing radiation therapy. Specifically, the radiation therapy system 10 emits radiation (therapeutic radiation). The therapeutic radiation emitted by the radiation therapy system 10 may be electromagnetic waves such as X-rays, or may be a particle beam such as an electron beam, a heavy particle beam, or a proton beam.

The radiation therapy system 10 captures a radiation image (for example, an X-ray fluoroscopic image). Further, the radiation therapy system 10 reconstructs a three-dimensional volume image based on the captured radiation image, and generates a computed tomography (CT) image. The radiation image captured by the radiation therapy system 10 and the CT image generated by the radiation therapy system 10 are used for specifying a position of an affected part and confirming the position of the affected part during emission of the radiation.

However, the generation of the CT image (reconstructed three-dimensional volume image) is not an essential function for the radiation therapy system 10 according to the present embodiment. For example, the radiation therapy system 10 may acquire a CT image generated by another device, and a user (for example, a doctor in charge of the radiation therapy) may specify the position of the affected part using the CT image.

The therapy plan apparatus 11 generates a therapy plan for the radiation therapy system 10 to emit the therapeutic radiation. Here, the therapy plan is a plan as to how the radiation therapy apparatus 13 is operated to emit the therapeutic radiation, and specifically, is information related to a content of control performed by the radiation therapy apparatus controller 12 on the radiation therapy apparatus 13. The therapy plan apparatus 11 includes, for example, at least a computer.

The radiation therapy apparatus controller 12 controls the radiation therapy apparatus 13 according to the therapy plan generated by the therapy plan apparatus 11 in order to emit the therapeutic radiation and capture the radiation image.

The radiation therapy apparatus 13 emits the therapeutic radiation and captures the radiation image according to the control of the radiation therapy apparatus controller 12. The radiation therapy apparatus 13 executes the therapy plan generated by the therapy plan apparatus 11 by emitting the therapeutic radiation according to the control of the radiation therapy apparatus controller 12.

FIG. 2 is a diagram illustrating an example of a configuration of the radiation therapy apparatus 13 according to Embodiment 1. For the sake of explanation, three-dimensional coordinates are illustrated. However, a coordinate system in FIG. 2 is an example, and is not limited thereto.

The radiation therapy apparatus 13 includes a rotation drive device 311, an O-ring 312, a traveling gantry 313, a swing mechanism (gimbal mechanism) 321, an emission unit 330, imaging radiation sources 341 and 342, sensor arrays 351, 361, and 362, and a patient support 381.

The emission unit 330 includes a radiation emitting device 331 and a multi leaf collimator (MLC) 332. The swing mechanism 321 and the imaging radiation sources 341 and 342 are independently provided on the traveling gantry 313.

The rotation drive device 311 supports, on a base, the O-ring 312 rotatably about a rotation axis A11, and rotates the O-ring 312 according to the control of the radiation therapy apparatus controller 12. The rotation axis A11 is an axis in a vertical direction.

The O-ring 312 is formed in a ring shape about a rotation axis A12, and supports the traveling gantry 313 rotatably about the rotation axis A12. The rotation axis A12 is an axis in a longitudinal direction of the patient support 381. The rotation axis A12 is an axis in a horizontal direction (that is, an axis perpendicular to the vertical direction), and is orthogonal to the rotation axis A11 at an isocenter P11. The rotation axis A12 is fixed with respect to the O-ring 312. That is, the rotation axis A12 rotates about the rotation axis A11 as the O-ring 312 rotates.

The traveling gantry 313 is formed in a ring shape about the rotation axis A12, and is disposed on an inner side of the O-ring 312 concentrically with respect to the O-ring 312. The radiation therapy apparatus 13 further includes a traveling driving device not shown, and the traveling gantry 313 rotates about the rotation axis A12 by power from the traveling driving device.

The traveling gantry 313 rotates itself to integrally rotate each part provided on the traveling gantry 313, such as the imaging radiation source 341 and the sensor array 361, the imaging radiation source 342 and the sensor array 362, and the sensor array 351.

The swing mechanism 321 is fixed on an inner side of the ring of the traveling gantry 313 and supports the emission unit 330 on the traveling gantry 313. The swing mechanism 321 supports the emission unit 330 in such a manner that an orientation can be changed, and changes the orientation of the emission unit 330 according to the control of the radiation therapy apparatus controller 12. Specifically, the swing mechanism 321 rotates the emission unit 330 about a pan axis A21. In addition, the swing mechanism 321 rotates the emission unit 330 about a tilt axis A22.

The pan axis A21 is an axis parallel to the rotation axis A12 and is fixed with respect to the traveling gantry 313. The swing mechanism 321 rotates the emission unit 330 about the pan axis A21, thereby causing the emission unit 330 to perform a swing operation in a right and left direction with respect to the rotation axis A12 (therefore, in a left and right direction with respect to a patient T11) .

The tilt axis A22 is an axis orthogonal to the pan axis A21, and is fixed with respect to the traveling gantry 313. The swing mechanism 321 rotates the emission unit 330 about the tilt axis A22, thereby causing the emission unit 330 to perform a swing operation in the direction of the rotation axis A12 (therefore, in an up and down direction with respect to the patient T11).

The emission unit 330 is disposed on an inner side of the traveling gantry 313 while being supported by the swing mechanism 321, and emits therapeutic radiation and imaging radiation.

The radiation emitting device 331 emits the therapeutic radiation to the affected part of the patient T11 according to the control of the radiation therapy apparatus controller 12.

The multi leaf collimator 332 opens and closes a leaf according to the control of the radiation therapy apparatus controller 12 to shield a part or all of the therapeutic radiation. Accordingly, the multi leaf collimator 332 adjusts an exposure field of the therapeutic radiation emitted to the patient T11. The multi leaf collimator 332 matches a shape of the exposure field of the therapeutic radiation with a shape of the affected part by shielding a part of the therapeutic radiation. The multi leaf collimator 332 adjusts intensity of the therapeutic radiation by blocking a part or all of the therapeutic radiation to adjust the exposure field.

Here, the leaf is a movable shielding object that is disposed to in such a manner that a part of the therapeutic radiation emitted by the emission unit 330 can be shielded. The leaf is implemented by, for example, a metal plate. The multi leaf collimator 332 includes a plurality of pairs of leaves arranged to be openable and closable, and adjusts the exposure field of the therapeutic radiation and adjusts the intensity by operating (moving) the leaves.

The imaging radiation source 341 emits imaging radiation (X-rays) toward the sensor array 361 according to the control of the radiation therapy apparatus controller 12. The imaging radiation source 342 emits the imaging radiation toward the sensor array 362 according to the control of the radiation therapy apparatus controller 12. The imaging radiation source 341 and the imaging radiation source 342 are fixed to the traveling gantry 313 independently of the emission unit 330 in orientations in which the radiation emitted by the imaging radiation source 341 and the radiation emitted by the imaging radiation source 342 are orthogonal to each other.

The sensor array 351 is fixed on an inner side of the ring of the traveling gantry 313 at a position onto which the therapeutic radiation from the radiation emitting device 331 hits facing the radiation emitting device 331. The sensor array 351 receives the therapeutic radiation, which is transmitted through the patient T11 and the like, to confirm an emitted position and record therapy. The term "receive" mentioned here refers to receiving the radiation.

The sensor array 361 is fixed on an inner side of the ring of the traveling gantry 313 at a position onto which the imaging radiation from the imaging radiation source 341 hits, facing the imaging radiation source 341. The sensor array 361 receives the imaging radiation, which is emitted from the imaging radiation source 341 and transmitted through the patient T11 and the like, to specify a position of the affected part.

The sensor array 361 receives the imaging radiation from the imaging radiation source 341 to obtain a radiation image.

The sensor array 362 is fixed on an inner side of the ring of the traveling gantry 313 at a position onto which the imaging radiation from the imaging radiation source 342 hits, facing the imaging radiation source 342. The sensor array 362 receives the imaging radiation, which is emitted from the imaging radiation source 342 and transmitted through the patient T11 and the like, to specify the position of the affected part.

The sensor array 362 receives the imaging radiation from the imaging radiation source 342 to obtain a radiation image. In particular, the radiation image from a direction different from that of a combination of the imaging radiation source 341 and the sensor array 361 is obtained by a combination of the imaging radiation source 342 and the sensor array 362.

A pair or two pairs of the imaging radiation source 341 and the sensor array 361, and the imaging radiation source 342 and the sensor array 362 simultaneously performs imaging while being rotated, thereby obtaining a CT image (for example, a cone beam CT (CBCT) image) of the patient T11 on the patient support 381.

The patient support 381 supports the patient T11 to be treated. In the present embodiment, the patient support 381 is a couch, and the patient T11 lies on the patient support 381.

The patient support 381 is provided such that the longitudinal direction thereof is in the direction of the rotation axis A12. The patient support 381 can move horizontally in various directions with the longitudinal direction thereof in the direction of the rotation axis A12. In particular, the patient support 381 is movable in the longitudinal direction thereof or a direction oblique to the longitudinal direction.

The traveling gantry 313 and the O-ring 312 correspond to an example of a rotation mechanism in the present embodiment. Specifically, the traveling gantry 313 supports the swing mechanism 321 rotatably about the axis (rotation axis A12) in the longitudinal direction of the patient support 381, and rotates itself to rotate the swing mechanism 321. The O-ring 312 supports the swing mechanism 321 to be rotatable about a second axis (rotation axis A11), and rotates itself to rotate the swing mechanism 321.

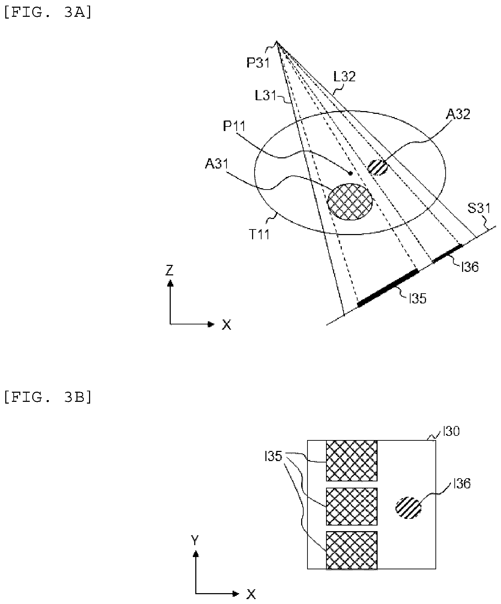

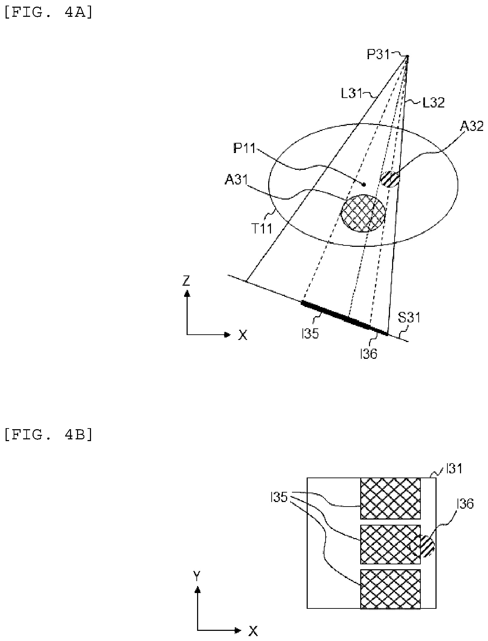

FIGS. 3A, 3B, 4A, and 4B are diagrams illustrating an example of the radiation image acquired by the radiation therapy apparatus 13 according to Embodiment 1. For the sake of explanation, the coordinate system illustrated in FIG. 2 is illustrated.

FIG. 3A illustrates a state in which the imaging radiation source 341 or the imaging radiation source 342 emits therapeutic radiation to the patient T1 from a point P31 in a range between a line L31 and a line L32. FIG. 4A illustrates a state in which the imaging radiation source 341 or the imaging radiation source 342 emits therapeutic radiation to the patient T11 after rotating the traveling gantry 313 about the isocenter P11 from the state in FIG. 3A.

A region A31 is a cross-section obtained by slicing a shape of an elliptical cone such as a spine, and represents a low transmittance region. The region A32 is a cross-section of soft tissue. An imaging surface S31 represents a cross-section (line) of the imaging surface of the sensor array 361 or the sensor array 362. A shade I35 represents a shade of the region A31 imaged on the imaging surface S31, and a shade I36 represents a shade of the region A32 imaged on the imaging surface S31.

FIG. 3B illustrates a radiation image I30 obtained by the sensor array 361 or the sensor array 362 receiving the radiation emitted to the patient T11 in the state in FIG. 3A. The radiation image I30 includes the shade I35 of the region A31 and the shade I36 of the region A32. The shade I35 and the shade I36 do not overlap with each other.

FIG. 4B illustrates a radiation image I31 obtained by the sensor array 361 or the sensor array 362 receiving the radiation emitted to the patient T11 in a state in FIG. 4A. The radiation image 131 includes the shade I35 of the region A31 and the shade I36 of the region A32. A part of the shade I36 overlaps with the shade 135.

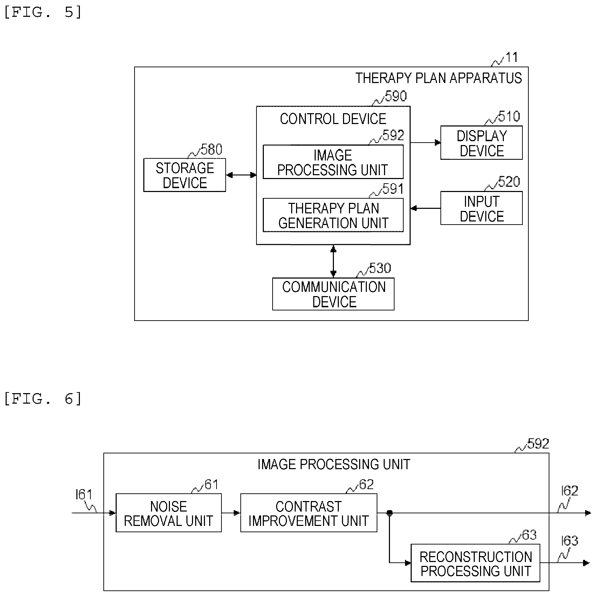

FIG. 5 is a diagram illustrating an example of a configuration of the therapy plan apparatus 11 according to Embodiment 1.

The therapy plan apparatus 11 includes a display device 510, an input device 520, a communication device 530, a storage device 580, and a control device 590.

The control device 590 is a central processing unit (CPU) or the like, and executes a program stored in the storage device 580. The control device 590 operates as a functional unit (module) that implements a specific function by executing processing according to the program. In the following description, when processing is described with the functional unit as a subject, it is indicated that the control device 590 executes the program for implementing the functional unit. The control device 590 according to the present embodiment functions as a therapy plan generation unit 591 and an image processing unit 592.

The display device 510 is, for example, a liquid crystal panel or an organic electro-luminescence (EL) panel, and displays various images such as a moving image, a still image, and a text (character). In particular, the display device 510 displays an image of the affected part (for example, a CT image of the affected part) and a content of the therapy plan generated by the therapy plan generation unit 591.

The input device 520 is, for example, a keyboard, a mouse, or a touch panel, and receives a user operation. In particular, the input device 520 receives a user operation for inputting various settings for generating the therapy plan. For example, in a state in which the display device 510 displays the image of the affected part, the input device 520 receives a user operation for inputting a region of the affected part and a region of an internal organ at risk in the image of the affected part. Further, the input device 520 receives a user operation for setting a target value and an allowable value of a radiation irradiation dose for the affected part and the internal organ at risk.

Here, the internal organ at risk is an internal organ in which the allowable value of the radiation dose is relatively small.

The communication device 530 communicates with the radiation therapy apparatus controller 12. In particular, the communication device 530 transmits the therapy plan generated by the therapy plan generation unit 591 to the radiation therapy apparatus controller 12.

The storage device 580 is a memory or the like and stores various kinds of information. In particular, the storage device 580 stores various pieces of setting information for generating the therapy plan and the therapy plan generated by the therapy plan generation unit 591.

The therapy plan generation unit 591 generates the therapy plan. According to the therapy plan generated by the therapy plan generation unit 591, the traveling gantry 313 continuously rotates. The radiation emitting device 331 continuously emits the therapeutic radiation according to the rotation of the traveling gantry 313. Further, the patient support 381 moves continuously according to the rotation of the traveling gantry 313. Further, the swing mechanism 321 adjusts the orientation of the radiation emitting device 331 according to the rotation of the traveling gantry 313.

In the therapy plan generated by the therapy plan generation unit 591, the multi leaf collimator 332 adjusts opening of the leaf according to the rotation of the traveling gantry 313.

By adjusting the shape and a size of the exposure field of the therapeutic radiation by the multi leaf collimator 332, it is possible to improve the accuracy of a dose distribution of the therapeutic radiation emitted by the radiation emitting device 331.

A position of the isocenter P11 relative to the patient T11 can be shifted by moving the patient support 381 not only in the longitudinal direction but also in the vertical and horizontal directions. Accordingly, a range in which the radiation emitting device 331 can emit the therapeutic radiation can be further expanded. Further, it is possible to further increase the accuracy of the dose distribution of the therapeutic radiation emitted by the radiation emitting device 331.

On the other hand, when the patient support 381 moves in a lateral direction of the patient T11, the patient T11 moves according to the movement, and position displacement of the patient T11 may be generated. Therefore, by moving the patient support 381 only in the longitudinal direction, the position displacement of the patient T11 can be reduced.

In the therapy plan, in addition to the movement of the patient support 381, the emission of the therapeutic radiation from the radiation emitting device 331, the orientation of the radiation emitting device 331 adjusted by the swing mechanism 321, and the rotation of the traveling gantry 313 around the rotation axis A11, the rotation of the O-ring 312 around the rotation axis A11 may be simultaneously controlled.

The O-ring 312 rotates the swing mechanism 321 around the rotation axis A11, thereby further expanding the range in which the radiation emitting device 331 can emit the therapeutic radiation. It is possible to further increase the accuracy of the dose distribution of the therapeutic radiation emitted by the radiation emitting device 331.

The image processing unit 592 performs image processing on the radiation image. The image processing executed by the image processing unit 592 will be described in detail later. The image processing unit 592 may be implemented using hardware dedicated to the image processing.

<Configuration Example and Operation Principle of Image Processing Unit>

FIG. 6 is a diagram illustrating an example of a functional configuration of the image processing unit 592 according to Embodiment 1.

The image processing unit 592 includes a noise removal unit 61, a contrast improvement unit 62, and a reconstruction processing unit 63.

The noise removal unit 61 executes noise removal processing on a signal I61 of the radiation image. For the noise removal processing, for example, a method of removing noise by comparing luminance values in one image (frame) and a method of removing noise by comparing a plurality of images (frames) are known. Since the methods for the noise removal processing are known techniques, a detailed description thereof will be omitted. By removing noise before the contrast is improved, it is possible to reduce an increase in the noise caused by the processing of the contrast improvement unit 62.

The contrast improvement unit 62 executes image processing for improving the contrast of the radiation image from which noise is removed. A signal I62 of the radiation image output, from the contrast improvement unit 62 may be output as it is, or may be output to the reconstruction processing unit 63. The display device 510 displays the signal I61 as a two-dimensional image.

The reconstruction processing unit 63 generates a CT image (three-dimensional volume image) by using a plurality of radiation images, and outputs a signal I63 of the CT image. The display device 510 displays the signal I63 as the CT image.

The image processing unit 592 can switch an image to be output in response to a request of the user.

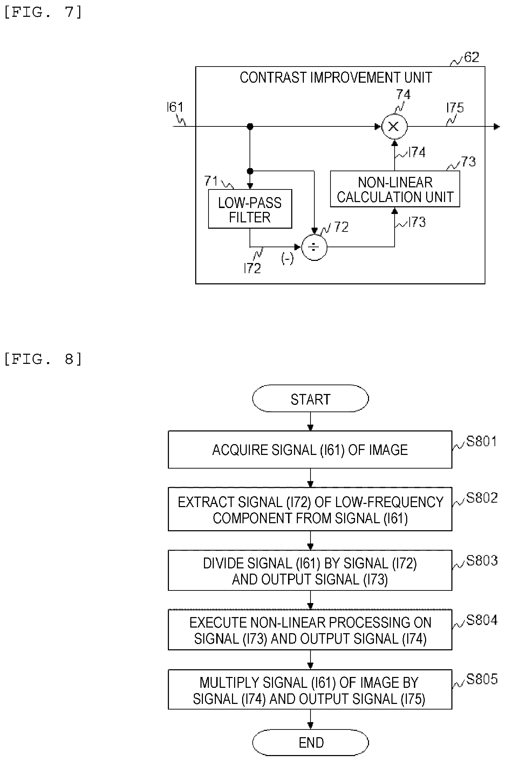

FIG. 7 is a diagram illustrating an example of a configuration of the contrast improvement unit 62 according to Embodiment 1. FIG. 8 is a flowchart illustrating an example of processing executed by the contrast improvement unit 62 according to Embodiment 1.

The contrast improvement unit 62 includes a low-pass filter 71, a divider 72, a non-linear calculation unit 73, and a multiplier 74. The contrast improvement unit 62 may include a functional unit that executes planarization processing and a functional unit that functions as a limiter.

The contrast improvement unit 62 acquires, from the noise removal unit 61, the signal I61 of the radiation image from which the noise is removed (step S801) .

The low-pass filter 71 extracts a signal I72 of a low-frequency component from the signal I61 of the radiation image from which the noise is removed, and outputs the signal I72 to the divider 72 (step S802) .

The divider 72 divides the signal I61 of the radiation image by the signal I72, and outputs a signal I73 obtained by the calculation to the non-linear calculation unit 73 (step S803). A luminance value of the signal I73 is 1 for a pixel at which luminance values of the signal I61 and the signal I72 are the same.

The non-linear calculation unit 73 executes non-linear processing on the signal I73, and outputs a signal I74 obtained by the calculation to the multiplier 74 (step S804) . Here, the non-linear processing is processing of converting the value of the input signal to a smaller value as the value of the input signal is larger and processing of converting the value of the input signal to a value not in a positive proportional relation to the value of the input signal.

The multiplier 74 multiplies the signal I61 of the radiation image by the signal I74 and outputs the signal I75 obtained by the multiplication (step S805) .

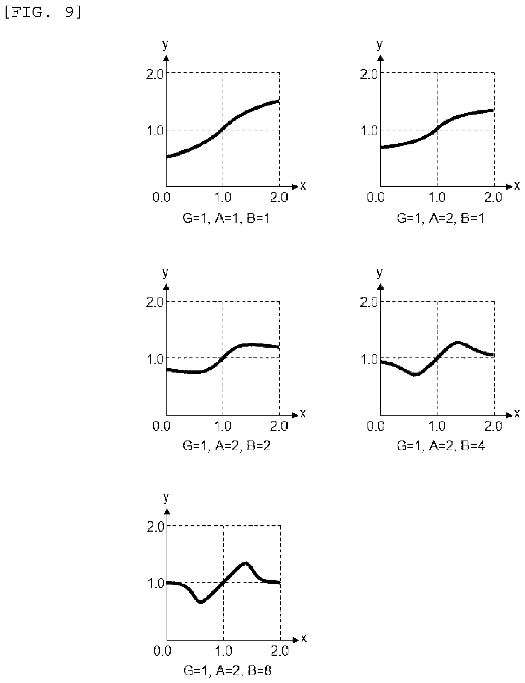

FIGS. 9, 10, and 11 are diagrams each illustrating an example of the non-linear processing executed by the non-linear calculation unit 73 according to Embodiment 1.

The non-linear calculation unit 73 executes a non-linear calculation f on a magnitude (x) of the luminance value included in the signal to calculate an output value y.

The non-linear calculation f can be defined as Equation (2), for example. Here, A, B, and G are constants. [Math. 2]

$$y = \frac{G * (x - 1)}{|A * (x - 1)|^B + 1} + 1 \,...\,(2)$$

In the non-linear calculation f expressed in Equation (2), when x is 1, y is 1. That is, the luminance value is not converted. If x is greater than 1, the value of y is less than or equal to x, and if x is less than 1, the value of y is greater than or equal to x. That is, the non-linear calculation f in Equation (2) has a characteristic of converting the value of x to a value close to 1 when the value of x is greatly deviated from 1.

By adjusting the constants A, B, and G, as illustrated in FIG. 9, the non-linear calculation having various change characteristics can be implemented. The values of the constants A, B, and G are illustrated below a graph.

The output value y changes in proportion to the constant G. When the constant G is increased, it is necessary to appropriately adjust since the noise increases. By adjusting the constant A, a change point of the output value y can be adjusted. When the constant A is increased, the change point of the output value y approaches x = 1. By adjusting the constant B, an attenuation amount (degree of approaching to 1) of the output value y can be adjusted. When the constant B is increased, the output value y rapidly approaches 1.

The non-linear calculation f can be defined as Equation (3). Here, A, B, and G are constants, and max is a maximum value function. [Math. 3]

$$y = x^{\frac{G}{max(x,1/x)^A - 1} + \frac{1}{B}} \quad \text{... (3)}$$

The non-linear calculation f in Equation (3) has a characteristic of Equation (4) with respect to the output value y.
[Math. 4]

$$y\left(\frac{1}{x}\right) = \frac{1}{y(x)} \quad \text{... (4)}$$

Therefore, when the luminance value of the signal I61 is smaller than the luminance value of the signal I72 (x < 1), or when the luminance value of the signal I61 is greater than the luminance value of the signal I72 (x > 1), a luminance change amount remains unchanged. Therefore, a natural image is obtained.

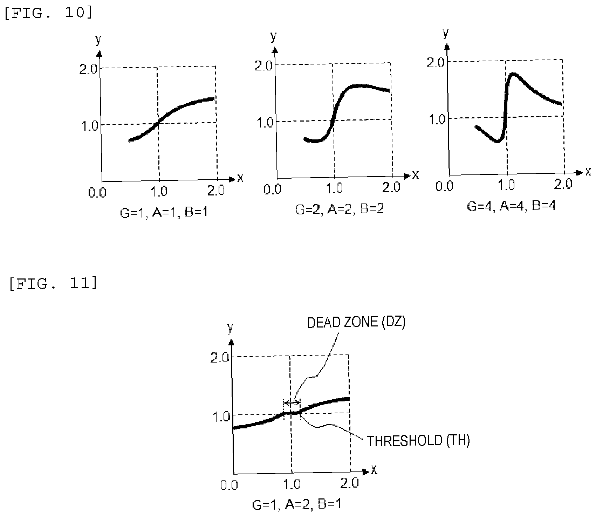

By adjusting the constants A, B, and G, as illustrated in FIG. 10, the non-linear calculation having various change characteristics can be implemented.

The non-linear calculation f can be defined as Equation (5). Here, A, B, and G are constants, max is a maximum value function, and TH is a threshold.

[Math. 5]

$$y = max(|\frac{G * (x - 1)}{|A * (x - 1)|^B + 1}| - TH, 0) * sign(x - 1)$$

$$+ 1 \quad \text{... (5)}$$

The non-linear calculation f in Equation (5) is expressed by, for example, a graph as illustrated in FIG. 11. As illustrated in FIG. 11, the non-linear calculation f has a dead zone (DZ) in a range of the value of x from - TH to TH, and the output value y is fixed at 1.

Decrease in the contrast of the radiation image is caused by two factors.

(1) A change amount (luminance gradient) of luminance values in a boundary between a region A and a region B around the region A is small.
(2) The region A and the region B around the region A have noise, and a luminance difference between the two regions is masked by the noise.

The factor (1) can be solved by the non-linear calculation f defined by Equations (2) and (3). Regarding the factor (2), in the non-linear calculation f in Equation (5), when signal intensity of the noise falls within the dead zone, the output value y is fixed to 1, and thus noise enhancement can be reduced.

The above non-linear calculation f is an example, and the invention is not limited thereto. For example, the non-linear calculation f may function as a limiter that prevents an absolute value of the luminance value from exceeding a certain value.

The non-linear calculation f may be set based on an empirical rule obtained by confirming an image quality. Alternatively, the value may be converted using a lookup table.

<Effect of Signal Processing according to Embodiment 1>

Next, an effect of the signal processing according to the present embodiment will be described in comparison with that of the technique described in PTL 1.

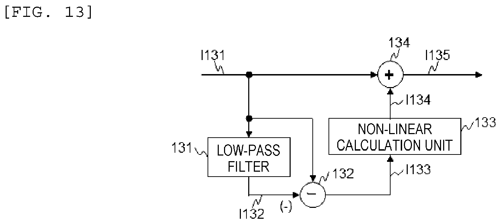

FIG. 13 is a diagram illustrating a configuration of the image processing apparatus described in PTL 1. Here, the image processing apparatus is simplified and described such that the image processing apparatus can be compared with the image processing unit 592 according to Embodiment 1.

The image processing apparatus described in PTL 1 includes a low-pass filter 131, a subtractor 132, a non-linear calculation unit 133, and an adder 134. An image signal I131 corresponds to the original image signal Sorg in PTL 1, a signal I132 corresponds to the blurred image signal Sus in PTL 1, a signal I133 corresponds to the signal (Sorg-Sus) in PTL 1, a signal 1134 corresponds to the conversion signal f(Sorg-Sus) in PTL 1, and a signal 1135 corresponds to a signal Sproc in PTL 1. The improvement coefficient β(Sorg) is simplified as "β is always 1" and is omitted in FIG. 13.

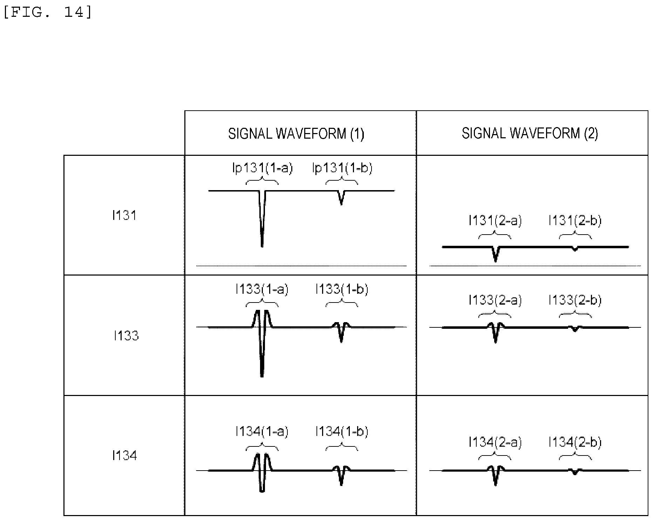

FIG. 14 is a diagram illustrating an example of a result of signal processing executed by the image processing apparatus described in PTL 1.

A signal waveform (1) in FIG. 14 (a column in a table) represents a signal waveform of a shade of a region not overlapping with a low transmittance region. For example, the signal waveform (1) is a signal waveform of the shade I36 in FIG. 3A. The signal waveform of the shade is observed with relatively high intensity.

A signal waveform (2) in FIG. 14 (a column in the table) represents a signal waveform of a shade of a region overlapping with the low transmittance region. For example, the signal waveform (2) is a signal waveform of a portion where the shade I35 and the shade I36 in FIG. 4A overlap with each other. Since the X-rays are greatly attenuated when passing through the low transmittance region, the signal waveform of the shade is observed with relatively low intensity.

A waveform Ip131(1-a) of the input signal I131 of the signal waveform (1) is a waveform corresponding to linear thin tissue that is difficult for the X-rays to transmit. Since the X-rays passing through the tissue greatly attenuate, the waveform has a large impulse shape in a negative direction. A waveform Ip131(1-b) of the input signal I131 of the signal waveform (1) is a waveform corresponding to linear thin tissue that is easy for the X-rays to transmit. Since the X-rays passing through the tissue do not attenuate much, the waveform has a small impulse shape in the negative direction.

A waveform Ip131(2-a) of the input signal I131 of the signal waveform (2) is a waveform corresponding to linear thin tissue that is difficult for the X-rays to transmit. Since the X-rays passing through the tissue greatly attenuate, the waveform has a large impulse shape in a negative direction. A waveform Ip131 (2-b) of the input signal I131 of the signal waveform (2) is a waveform corresponding to linear thin tissue that is easy for the X-rays to transmit. Since the X-rays passing through the tissue do not attenuate much, the waveform has a small impulse shape in the negative direction. Signal intensity of the input signal I131 of the signal waveform (2) is smaller than signal intensity of the input signal I131 of the signal waveform (1) due to the attenuation of the X-rays at the low transmittance region.

A waveform Ip133(1-a) of the signal I133 of the signal waveform (1) is a waveform after the subtractor 132 performs processing on the waveform Ip131(1-a). A waveform Ip133(1-b) of the signal I133 of the signal waveform (1) is a waveform after the subtractor 132 performs processing on the waveform Ip131(1-b). A waveform Ip133(2-a) of the signal I133 of the signal waveform (2) is a waveform after the subtractor 132 performs processing on the waveform Ip131(2-a). A waveform Ip133(2-b) of the signal I133 of the signal waveform (2) is a waveform after the subtractor 132 performs processing on the waveform Ip131(2-b).

A waveform Ip134(1-a) of the signal I134 of the signal waveform (1) is a waveform after the non-linear calculation unit 133 performs processing on the waveform Ip131(1-a). A waveform Ip134(1-b) of the signal I134 of the signal waveform (1) is a waveform after the non-linear calculation unit 133 performs processing on the waveform Ip131(1-b). A waveform Ip134(2-a) of the signal I134 of the signal waveform (2) is a waveform after the non-linear calculation unit 133 performs processing on the waveform Ip131(2-a). A waveform Ip134 (2-b) of the signal I134 of the signal waveform (2) is a waveform after the non-linear calculation unit 133 performs processing on the waveform Ip131(2-b).

Intensity after the non-linear processing greatly changes according to intensity of an input signal (magnitude of a luminance value). Even though a parameter for the non-linear processing is adjusted such that a peak value of the intensity of the waveform Ip134(1-a) is substantially equal to a peak value of the intensity of the waveform Ip134(1-b), a peak value of the intensity of the waveform Ip134(2-a) is not substantially equal to a peak value of the intensity of the waveform Ip134 (2-b). That is, in the related art, there is a problem that the contrast improvement effect greatly differs for the shade of the region overlapping the low transmittance region and the shade of the region not overlapping with the low transmittance region.

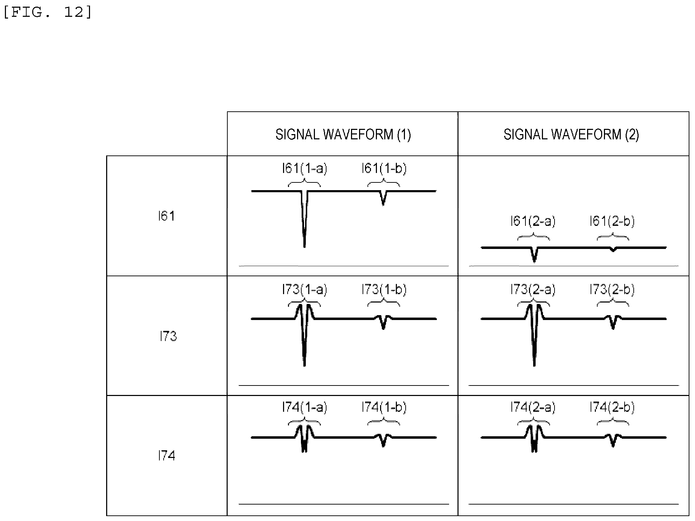

FIG. 12 is a diagram illustrating an example of a result of the signal processing executed by the contrast improvement unit 62 according to Embodiment 1.

A signal waveform (1) in FIG. 12 (a column in a table) represents a signal waveform of the shade of the region not overlapping with the low transmittance region. A signal waveform (2) in FIG. 12 (a column in the table) represents a signal waveform of the shade of the region overlapping with the low transmittance region.

A waveform Ip73(1-a) of the signal I73 of the signal waveform (1) is a waveform after the divider 72 performs processing on a waveform Ip61(1-a). A waveform Ip73(1-b) of the signal I73 of the signal waveform (1) is a waveform after the divider 72 performs processing on a waveform Ip61(1-b). A waveform Ip73(2-a) of the signal I73 of the signal waveform (2) is a waveform after the divider 72 performs processing on a waveform Ip61(2-a). A waveform Ip73(2-b) of the signal I73 of the signal waveform (2) is a waveform after the divider 72 performs processing on a waveform Ip61(2-b).

Signal intensity of the signal I73, which is the low-frequency component of the signal I61, is proportional to signal intensity of the signal I61. Therefore, the signal intensity of the signal I73 is substantially the same regardless of a magnitude of the signal intensity of the signal I61. Therefore, waveforms having the same signal intensity are output from the divider 72 regardless of the presence or absence of overlapping with the low transmittance region.

A waveform Ip74(1-a) of the signal I74 of the signal waveform (1) is a waveform after the non-linear calculation unit 73 performs processing on the waveform Ip61(1-a). A waveform Ip74(1-b) of the signal I74 of the signal waveform (1) is a waveform after the non-linear calculation unit 73 performs processing on the waveform Ip61(1-b). A waveform Ip74(2-a) of the signal I74 of the signal waveform (2) is a waveform after the non-linear calculation unit 73 performs processing on the waveform Ip61(2-a). A waveform Ip74 (2-b) of the signal I74 of the signal waveform (2) is a waveform after the non-linear calculation unit 73 performs processing on the waveform Ip61(2-b).

Since the signal intensity of the signal I73 input to the non-linear calculation unit 73 is almost the same regardless of the presence or absence of overlapping with the low transmittance region, the same calculation result is obtained.

According to the present embodiment, the same contrast improvement effect can be obtained for both the shade of the region not overlapping with the low transmittance region and the shade of the region overlapping with the low transmittance region.

The invention can be implemented by a program code of software implementing the functions according to the embodiment. In this case, a storage medium recording the program code is provided to a system or a device, and a computer (or CPU or MPU) of the system or the device reads the program code stored in the storage medium. In this case, the program code itself read from the storage medium implements the functions according to the above embodiment, and the program code itself and the storage medium storing the program code constitute the invention. Examples of the storage medium for supplying such a program code include a flexible disc, a CD-ROM, a DVD-ROM, a hard disc, an optical disc, a magneto-optical disc, a CD-R, a magnetic tape, a nonvolatile memory card, and a ROM.

An operating system (OS) or the like running on the computer may perform a part or all of actual processing based on an instruction of the program code so as to implement the functions according to the above embodiment by the processing. After the program code read from the storage medium is written in a memory of the computer, the CPU or the like of the computer may perform a part or all of the actual processing based on the instruction of the program code so as to implement the functions according to the above embodiment by the processing.

Further, by distributing, via a network, the program code of the software implementing the functions according to the embodiment, the program code may be stored in a storage device such as a hard disc or a memory of the system or the device or in a storage medium such as a CD-RW or a CD-R, and the system or the computer of the device (a CPU, a micro-processing unit (MPU), a graphics processing unit (GPU), or the like) may read and execute the program code stored in the storage device or the storage medium during use.

Finally, it is necessary to understand that the processes and techniques described herein are not inherently relevant to any particular device and may be implemented by any suitable combination of components. Further, various types of devices for general purpose may be used in accordance with the teachings described herein. It may prove that it is beneficial to construct a specialized device to execute the steps of the method described herein. Various inventions can be made by appropriately combining a plurality of the components described in the embodiment. For example, several components may be deleted from all of the components described in the embodiment. Further, the components in different embodiments may be appropriately combined. The invention is described in relation to specific examples, which are intended in all respects to be illustrative rather than restrictive. A person skilled in the art will recognize that there.are numerous combinations of hardware, software, and firmware that are suitable for implementing the invention. For example, the described hardware may be implemented by an application specific integrated circuit (ASIC), or a field-programmable gate array (FPGA), and the described software may be implemented by a wide range of programs or script languages such as an assembler, C/C++, perl, Shell, PHP, Python, and Java.

Further, in the above embodiment, control lines and information lines are considered to be necessary for description, and all control lines and information lines are not necessarily illustrated in the product. All of the configurations may be connected to each other.

In addition, for a person skilled in the art, other implementations of the invention will be apparent in consideration of the specification and embodiments of the invention disclosed herein. Various aspects and/or components of the above embodiment can be used individually or in any combination in a computerized storage system having a function of managing data.

**Claims**

1.  A radiation image processing apparatus for executing image processing on an image obtained by emitting radiation to an object, the radiation image processing apparatus comprising:

    a calculation device;
    a storage device connected to the calculation device; and
    an interface connected to the calculation device, wherein
    the radiation image processing apparatus

    acquires an input signal of the image, and
    executes contrast improvement processing on the input signal of the image to improve a contrast of the image, and

the contrast improvement processing includes

processing of extracting a low-frequency component of the input signal of the image to generate a first signal of the image,
processing of dividing the input signal of the image by the first signal of the image to generate a second signal of the image,
processing of executing non-linear processing on the second signal of the image to generate a third signal of the image, and
processing of outputting an output signal of the image obtained by multiplying the input signal of the image by the third signal of the image.

2. The radiation image processing apparatus according to claim 1, wherein

noise removal processing is executed on the input signal of the image, and
the contrast improvement processing is executed on the input signal of the image on which the noise removal processing is executed.

3. An image processing method to be executed by a radiation image processing apparatus and for an image obtained by emitting radiation to an object,

the radiation image processing apparatus including

a calculation device,
a storage device connected to the calculation device, and
an interface connected to the calculation device,

the image processing method comprising:

a first step of acquiring an input signal of the image by the calculation device; and
a second step of executing contrast improvement processing on the input signal of the image by the calculation device to improve a contrast of the image, wherein

the second step includes

a step of extracting a low-frequency component of the input signal of the image to generate a first signal of the image by the calculation device,
a step of dividing the input signal of the image by the first signal of the image to generate a second signal of the image by the calculation device,
a step of executing non-linear processing on the second signal of the image to generate a third signal of the image by the calculation device, and
a step of outputting an output signal of the image obtained by multiplying the input signal of the image by the third signal of the image by the calculation device.

4. The image processing method according to claim 3, wherein

the first step includes a step of executing noise removal processing on the input signal of the image by the calculation device, and
in the second step, the calculation device executes the contrast improvement processing on the input signal of the image on which the noise removal processing is executed.

5. A system comprising:

an image generation apparatus configured to emit radiation to an object to acquire an image; and
a radiation image processing apparatus configured to execute image processing on the image, wherein
the radiation image processing apparatus

acquires an input signal of the image, and

executes contrast improvement processing on the input signal of the image to improve a contrast of the image, and

the contrast improvement processing includes

processing of extracting a low-frequency component of the input signal of the image to generate a first signal of the image,

processing of dividing the input signal of the image by the first signal of the image to generate a second signal of the image,

processing of executing non-linear processing on the second signal of the image to generate a third signal of the image, and

processing of outputting an output signal of the image obtained by multiplying the input signal of the image by the third signal of the image.

6. The system according to claim 5, wherein the radiation image processing apparatus

executes noise removal processing on the input signal of the image, and

executes the contrast improvement processing on the input signal of the image on which the noise removal processing is executed.

[FIG. 1]

[FIG. 2]

[FIG. 3A]

[FIG. 3B]

[FIG. 4A]

[FIG. 4B]

[FIG. 5]

```
                                                    ⌇11
┌─────────────────────────────────────────────────────────────┐
│                              THERAPY PLAN APPARATUS           │
│                    ⌇590                                        │
│              CONTROL DEVICE                                    │
│                              ⌇592          ┌──────────┐       │
│    ⌇580                  ┌──────────────┐   │  ⌇510    │       │
│                         │    IMAGE     │   │ DISPLAY  │       │
│ ┌──────────┐            │  PROCESSING  │──▶│ DEVICE   │       │
│ │ STORAGE  │◀──────────▶│    UNIT      │   └──────────┘       │
│ │ DEVICE   │            ├──────────────┤          ⌇520        │
│ └──────────┘            │     ⌇591     │   ┌──────────┐       │
│                         │ THERAPY PLAN │   │  INPUT   │       │
│                         │  GENERATION  │◀──│ DEVICE   │       │
│                         │     UNIT     │   └──────────┘       │
│                         └──────────────┘                      │
│                                 ▲   ⌇530                       │
│                                 ▼                             │
│                         ┌──────────────┐                      │
│                         │COMMUNICATION │                      │
│                         │   DEVICE     │                      │
│                         └──────────────┘                      │
└─────────────────────────────────────────────────────────────┘
```

[FIG. 6]

```
                                                          ⌇592
┌──────────────────────────────────────────────────────────────┐
│               IMAGE PROCESSING UNIT                           │
│                                                                │
│ I61    ⌇61           ⌇62                                 I62   │
│    ┌──────────┐ ┌──────────────┐                              │
│───▶│  NOISE   │▶│   CONTRAST   │●──────────────────────────▶  │
│    │ REMOVAL  │ │ IMPROVEMENT  ││                             │
│    │  UNIT    │ │    UNIT      ││          ⌇63          I63   │
│    └──────────┘ └──────────────┘│   ┌──────────────┐          │
│                                 └──▶│RECONSTRUCTION│────────▶ │
│                                     │  PROCESSING  │          │
│                                     │    UNIT      │          │
│                                     └──────────────┘          │
└──────────────────────────────────────────────────────────────┘
```

17

[FIG. 7]

[FIG. 8]

[FIG. 9]

G=1, A=1, B=1

G=1, A=2, B=1

G=1, A=2, B=2

G=1, A=2, B=4

G=1, A=2, B=8

[FIG. 10]

G=1, A=1, B=1     G=2, A=2, B=2     G=4, A=4, B=4

[FIG. 11]

DEAD ZONE (DZ)

THRESHOLD (TH)

G=1, A=2, B=1

[FIG. 12]

| | SIGNAL WAVEFORM (1) | SIGNAL WAVEFORM (2) |
|---|---|---|
| I61 | I61(1-a) I61(1-b) | I61(2-a) I61(2-b) |
| I73 | I73(1-a) I73(1-b) | I73(2-a) I73(2-b) |
| I74 | I74(1-a) I74(1-b) | I74(2-a) I74(2-b) |

[FIG. 13]

[FIG. 14]

## INTERNATIONAL SEARCH REPORT

| International application No. |
| --- |
| **PCT/JP2022/001472** |

**A. CLASSIFICATION OF SUBJECT MATTER**

*A61B 6/00*(2006.01)i
FI:   A61B6/00 350M; A61B6/00 370

According to International Patent Classification (IPC) or to both national classification and IPC

**B. FIELDS SEARCHED**

Minimum documentation searched (classification system followed by classification symbols)

A61B6/00-6/14; G06T5/00-5/50

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
| --- | --- | --- |
| A | JP 2020-065614 A (HITACHI LTD) 30 April 2020 (2020-04-30)<br>in particular, paragraphs [0053]-[0059], [0085]-[0108], fig. 6-10 | 1-6 |
| A | JP 2017-107260 A (SEIKO EPSON CORP) 15 June 2017 (2017-06-15)<br>in particular, paragraphs [0030]-[0063], fig. 2-3 | 1-6 |
| A | JP 2016-154766 A (KONICA MINOLTA INC) 01 September 2016 (2016-09-01)<br>in particular, paragraphs [0066]-[0071], fig. 11 | 1-6 |
| A | JP 2010-187394 A (SONY CORP) 26 August 2010 (2010-08-26)<br>in particular, paragraphs [0041]-[0044], fig. 6 | 1-6 |

☐ Further documents are listed in the continuation of Box C.     ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **04 March 2022** | **22 March 2022** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
**Information on patent family members**

International application No.

**PCT/JP2022/001472**

| Patent document cited in search report | | | Publication date (day/month/year) | Patent family member(s) | Publication date (day/month/year) |
|---|---|---|---|---|---|
| JP | 2020-065614 | A | 30 April 2020 | (Family: none) | |
| JP | 2017-107260 | A | 15 June 2017 | (Family: none) | |
| JP | 2016-154766 | A | 01 September 2016 | US 2016/0253785 A1 in particular, paragraphs [0116]-[0123], fig. 11 | |
| JP | 2010-187394 | A | 26 August 2010 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021101326 A

- JP 2002074356 A

**Non-patent literature cited in the description**

- **GUANG DENG.** A Generalized Unsharp Masking Algorithm. *IEEE TRANSACTIONS ON IMAGE PROCESSING,* May 2011, vol. 20 (5